(19)

Europäisches
Patentamt
European
Patent Office
Office européen
des brevets

(11) **EP 2 503 002 A1**

(12) **EUROPEAN PATENT APPLICATION**

(43) Date of publication:
    **26.09.2012 Bulletin 2012/39**

(51) Int Cl.:
    *C12P 5/02* [(2006.01)]     *C10J 3/00* [(2006.01)]

(21) Application number: **11159200.2**

(22) Date of filing: **22.03.2011**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**

(71) Applicant: **Ludan Renewable Energy B.V.
    3115 JG Schiedam (NL)**

(72) Inventor: **Jansen, Franciscus Cornelis
    2671 ZR Naaldwijk (NL)**

(74) Representative: **Swinkels, Bart Willem
    Nederlandsch Octrooibureau
    J. W. Frisolaan 13
    2517 JS Den Haag (NL)**

(54) **Methane, biogas or syngas production using Pennisetum feedstock**

(57)    The present invention concerns the field of methane, biogas and/or syngas production using biomass feedstock. More in particular, the invention concerns a new feedstock material for methane, biogas and/or syngas production. It is an objective of the present invention to provide a process of energy recovery from biomass wherein overall efficiency is improved. The present inventors have found that this objective can be met by using plant material derived from the genus *Pennisetum,* especially from *Pennisetum hybridum,* as the feedstock in biogas or methane production or in gasification.

EP 2 503 002 A1

**Description**

**FIELD OF THE INVENTION**

The present invention concerns the field of methane, biogas and/or syngas production using biomass feedstock. More in particular, the invention concerns a new feedstock material for methane, biogas and/or syngas production.

**BACKGROUND**

Since fossil fuels are non-renewable resources, adequate supplies of energy and organic feedstocks need to be secured for the future. A transition to sustainable resources requires new technologies for the construction of improved feedstocks, the design of efficient processes to convert the feedstocks into valuable products, and/or the design of products that efficiently utilize an altered substrate spectrum. This transformation should create benefits such as decreased pollution from energy production and use, increased sustainability through the utilization of renewable natural resources, decreased dependence on foreign country's raw materials, and an increase in local economies and markets involved in the production of new substrates.

Plant biomass is one sustainable resource that can help meet future feedstock requirements. The use of plants as substrates for energy and organic feedstock takes advantage of existing large-scale agricultural production, uses energy from the sun to incorporate carbon dioxide into plants via photosynthesis, and has fewer environmentally hazardous by-products. By using photosynthesis, plants make the carbon dioxide removed from the air available for the production of energy, chemicals, and agricultural products. Energy production from biomass is regarded as being "$CO_2$-neutral", since the amount of $CO_2$ released by combustion of a given amount of the biomass corresponds to the amount of $CO_2$ which was originally taken up from the atmosphere during the build-up of that biomass.

Biogas, which generally relates to methane and $CO_2$, has been produced from a variety of sources such as, animal wastes (manures), agricultural wastes, industrial wastes, municipal wastes, and sewage wastes. Biogas generation from such wastes involve anaerobic digestion or fermentation of the biological material in the presence of suitable microbes. Some of these biogas generation processes involve certain pretreatments, such as, for example, treating a polysaccharide material such as cellulose, hemicellulose and lignocellulose by acid hydrolysis.

Microbial conversion of organic matter to methane has become an attractive option for treatment of wastes for energy recovery. Some of the characteristic features of anaerobic digestion with microbes are: (i) bacteria involved in biogas production process are anaerobes and slow growing; (ii) some degree of metabolic specialization is observed in these anaerobic microorganisms; and (iii) that most of the free energy present in the substrate is captured in the terminal product methane. Because of the slow growth and low energy requirement for these microbes, less microbial biomass is produced and therefore, disposal of sludge after the digestion may not be an issue.

Alternatively 'syngas' can be produced in a process referred to as gasification. Gasification is a process that converts carbonaceous materials, such as coal, petroleum, biofuel, or biomass, into carbon monoxide and hydrogen by reacting the raw material at high temperatures with a controlled amount of oxygen and/or steam. The resulting gas mixture is called synthesis gas or syngas and is itself a fuel. Gasification is a method for extracting energy from many different types of organic materials. The advantage of gasification is that using the syngas is potentially more efficient than direct combustion of the original fuel because it can be combusted at higher temperatures or even in fuel cells, so that the thermodynamic upper limit to the efficiency defined by Carnot's rule is higher or not applicable. Syngas may be burned directly in internal combustion engines, used to produce methanol and hydrogen, or converted via the Fischer-Tropsch process into synthetic fuel. Gasification can also begin with materials that are not otherwise useful fuels, such as biomass or organic waste. A variety of plant feed materials have been used as feedstock material for methanogenesis and/or gasification processes. For example maize, willow, triticale, Pawlonia, sugarbeet, etc. Usually, high water content biomasses, such as silages from cereals or sugarbeet are utilized for biogas generation, while low humidity biomasses, such as willow or Pawlonia, are usually utilized for gasification processes. Only few biomasses can be valorized in both ways

In the past, generally, the cost basis of most bioenergy methodologies have not been competitive with that of traditional fossil fuels. However, with the supply of fossil fuels decreasing with an increasing global demand for fossil fuels, production of renewable fuels from biomass has become an attractive and viable option. Nevertheless, the efficiency of energy recovery from biomass needs to be increased by increasing production rates, reducing production costs, reducing fermentation time, while increasing the yield per unit weight of biomass.

It is an objective of the present invention to provide a process of energy recovery from biomass wherein overall efficiency is improved.

**SUMMARY OF THE INVENTION**

The present invention, generally stated, concerns the use of biomass material originating from *Pennisetum,* especially from *Pennisetum hybridum,* in both fermentative as well as synthetic biogas production. The present inventors have surprisingly found that the use of a feedstock based on this biomass material results in high overall efficiency of energy recovery, both in digestive biogas/methane processes as well as in gasification processes.

In the context of the present invention, 'overall efficiency' generally relates to economical factors in generation of methane gas or syngas from organic substrate, which factors include agricultural yield of the biomass, quality of the biomass, retention time of the substrate in the digestion process, methane yield per unit biomass or syngas yield per unit biomass, methane content per unit volume of biogas and other factors that affect the overall cost of methane or syngas production.

One particular strategy of in increasing the efficiency is to find a suitable plant material with a high methane yield potential and/or a high syngas yield potential. For a crop to be suitable as a feedstock it is however also required that the crop can be grown in high yields, ideally in the geographical region where its use as a feedstock is desired. Hence, ideally a crop should have high $kg/m^2$ yields as well as high $m^3/kg$ yields.

The invention is based, in one part, on the surprising discovery that *Pennisetum,* especially *Pennisetum hybridum,* can be grown with high yields (kg of biomass per surface area unit of agricultural land) in mild climates, such as those of western Europe and northern America, which are quite distinct from the climate of this crop's original environment.

The invention is based, in another part, on the discovery that the *pennisetum* plant material is a suitable substrate for efficient methanogenic digestion as well gasification. Data provided in the appended examples demonstrate that methane yields generated with *pennisetum* plants as a feedstock in mesophilic and theremophilic digestion, is sufficient to render large-scale use of this crop for energy generation economically feasible.

**DETAILED DESCRIPTION OF THE INVENTION**

Thus, a first aspect of the present invention concerns a process for producing methane gas or biogas from solid biomass material, the process comprising the steps of: (i) providing biomass; (ii) preparing a feedstock slurry containing microbially fermentable components by combining the biomass with an aqueous phase and, optionally, subjecting the biomass to a pre-treatment resulting in reduced resistance to enzymatic degradation; and (iiia) incubating the feedstock slurry at a temperature of between 55 and 60 °C and under an oxygen depleted atmosphere, in the presence of methane-producing thermophilic microorganisms and collecting the methane gas released from the fermentation feedstock; or (iiib) incubating said feedstock slurry at a temperature of between 36 and 42 °C and under an oxygen depleted atmosphere, in the presence of methane-producing mesophilic microorganisms and collecting the methane gas released from the slurry; **characterized** in that the biomass comprises matter derived from a plant belonging to the genus *Pennisetum,* especially *Pennisetum hybridum.*

The term biomass in general is understood to refer to living matter formed by photosynthesis, for example plants, trees, grasses etc., as well as useful materials and wastes derived from living sources including animals and humans in time scales that are small compared to those for the formation of fossil fuels. This definition clearly excludes traditional fossil fuels, since although they are also derived from plant (coal) or animal (oil and gas) life, it has taken millions of years to convert them to their current form. Examples of useful materials and wastes derived from living sources include municipal wastes, as well as finished products, recycled materials, and wastes from agriculture, forestry, construction, and human and animal habitat. Thus, biomass includes any of wood, cellulose, hemicellulose, lignin, lignocellulosic materials, or mixtures thereof, paper, as well as wastes and residues from forests, animals, and humans, including municipal waste, that are at least partially organic in their makeup, and any plant material or residue of a plant, whether such plant or residue is living or not.

In accordance with the present invention the biomass at least comprises matter derived from a plant belonging to the genus *Pennisetum.* This is a genus of grasses in the family Poaceae, native to tropical and warm temperate regions of the world, in particular East Africa, tropical Africa, Middle East and SW Asia.

In a particularly preferred embodiment of the invention, the biomass is derived from a plant belonging to the species *Pennisetum hybridum. Pennisetum hybridum* is also referred to as 'maralfalfa'. *Pennisetum hybridum* is also sometimes referred to as *Pennisetum violaceum.* In the context of the present invention these terms are deemed synonymous. *Pennisetum hybridum* has been found to provide the best combination of agricultural yields on the one hand, even when grown under conditions not common to the geographical areas from which the crop originates, and methane yield potential on the other hand, which together determine the suitability of a crop to be used as feedstock for biogas production.

As will be understood by those skilled in the art, the present invention also encompasses the use of *Pennisetum* or *Pennisetum hybridum* mutants, both naturally occurring and those that have been selected using classical marker assisted breeding techniques and genetic engineering techniques. *Pennisetum* also refers to *Pennisetum* hybrids.

In accordance with the present invention any part of the plant may be used. In a preferred embodiment of the invention, a process as defined herein before is provided, wherein the biomass is derived from the stem and leaves. In another preferred embodiment the entire plant is used as biomass.

As will be understood by those skilled in the art, the present process may employ the *Pennisetum* or *Pennisetum hybridum* plant matter as the only source of biomass. However, embodiments are also envisaged wherein the *Pennisetum* or *Pennisetum hybridum* plant material is used in combination with other, common, biomass materials, such as, in particular, (1) agricultural wastes, such as corn stalks, straw, seed hulls, sugarcane leavings, bagasse, nutshells, and manure from cattle, poultry, and hogs; (2) woody materials, such as wood or bark, sawdust, timber slash, and mill scrap; (3) municipal waste, such as waste paper and yard clippings; (4) other energy crops, such as silages from cereals, poplars, willows, corn, soybean, etc.; and (5) coal, peat moss, and the like.

In an embodiment, all biomass used to prepare the feedstock slurry is derived from a *Pennisetum* plant, especially *Pennisetum hybridum,* although embodiments wherein other materials are admixed with the *Pennisetum* derived biomass are also envisaged. If other biomass material is used, it is preferred that at least 50 wt%, more preferably at least 75 wt%, more preferably at least 85 wt%, most preferably at least 90wt% of the total solids weight of the biomass is a plant material derived from *Pennisetum,* most preferably from a plant material derived from *Pennisetum hybridum.* Furthermore, the ratio of *Pennisetum* plant material, preferably *Pennisetum hybridum* plant material, to non- *Pennisetum* plant material typically exceeds 1:1, preferably 2:1, more preferably 5:1, and most preferably 10:1..

As used herein the term 'feedstock slurry' refers to a mixture containing water and microbially fermentable components derived from the biomass described above. In order to prepare such a feedstock slurry the biomass is combined with an aqueous composition. Several further pre-treatment steps may be employed which are typically aimed at increasing the biogas formation rate and biogas yield. These pre-treatment steps include for example mechanical treatments to reduce the size of the biomass material as well as mechanical, physical, chemical and/or enzymatic treatments aimed at reducing resistance to enzymatic degradation by the fermenting microorganisms.

In a preferred embodiment of the invention, the feedstock slurry is prepared by suspending the biomass in an aqueous composition to form a slurry. The effective percentage of solids typically is an amount which enables the balancing of microorganism growth and acid production. Typically, the feedstock slurry comprise less than 30% wt. solids, preferably it comprises 0.1-20% wt. solids, more preferably 10-15% wt. Typically, the aqueous composition may comprise other dissolved or suspended components such as manure or sludge. In a preferred embodiment of the invention the biomass and the aqueous composition are combined in such ratios that the feedstock slurry comprises less than 30 wt%, preferably 0.1-20 %wt., more preferably 10-15 wt% wt of matter derived from a plant of the genus *Pennisetum,* more preferably from *Pennisetum hybridum.*

In one embodiment of the invention, the process comprises reducing the particle size of the solid matter. The native physical form of the biomass used in the present invention, will generally necessitate or at least make it desirable to carry out comminuting of said solid matter, e.g. by milling, abrading, grinding, crushing, chopping or the like, to some extent in order to obtain particles of sufficiently small size and/or sufficiently high surface area to mass ratio to enable degradation of the material at a satisfactory rate and to a satisfactory extent. The particle size reduction can be performed either prior to, after, or during preparation of the feedstock slurry by employing an in-line or immersed abrader, classifier, mill, high shear mixer, grinder, homogenizer or other particle size reducer known to those of ordinary skill in the art. No particular particle size is required for the solid matter in the feedstock slurry; however, smaller particle sizes are preferred as smaller particles are generally bioconverted more quickly than larger particles.

In a preferred embodiment of the invention the average particle size of the solid matter of the feedstock slurry is within the range of 1 mm - 10 mm, more preferably within the range of 2 mm - 5 mm.

As is generally understood by those skilled in the art, in biomass disintegration technologies two primary effects are to be taken into account, i.e. the rate of degradation and the extent of degradation of organic matter. Faster (anaerobic) degradation saves digester volume. More complete degradation of the organic matter results in higher biogas potential and less residual solids production. The matter derived from a plant belonging to the genus *Pennisetum,* as well as the optional other biomass material used for preparing the feedstock slurry typically include significant amounts of lignocelluloses. Lignocelluloses are composed mainly of cellulose, hemicellulose and lignin. Cellulose chains are packed by hydrogen bonds in so-called 'elementary and microfibrils'. These fibrils are attached to each other by hemicelluloses as well as other polymers such as pectin. The cellulose and hemicellulose are cemented together by lignin. Lignin is responsible for integrity, structural rigidity, and prevention of swelling of lignocelluloses. It is believed that lignin may function as a physical barrier to the direct bioconversion (e.g. by fermenting microorganisms) of cellulose and hemicellulose in lignocellulosic materials which have not been subjected to some kind of pretreatment process to disrupt the structure of lignocellulose and to achieve at least partial separation of the material into cellulose, hemicellulose and lignin. For increased efficiency of the fermentation step, in terms of rate and/or extent of conversion of the organic matter, it is preferred that breakdown of complex macromolecules into their basic components is performed beforehand, eg. by mechanical disintegration methods, chemical treatment, ultrasonic technique, thermal pre-treatment and enzymatic pre-treatment.

Hence, in a preferred embodiment, a pretreatment process is performed in order to improve formation and yield of biogas. A number of methods exist for this according to the prior art and primarily in the field of raw materials recovery from renewable raw materials. Various embodiments, involving (combinations of) mechanical pre-treatment (milling, grinding), physical pretreatment (vapor explosion method, hot water treatment, thermopressure hydrolysis), chemical pretreatment (acidic treatment, alkaline treatment, peroxide treatment, organosolvent treatment, wet oxidation) and enzymatic methods are envisaged in the context of the present invention. In a preferred embodiment, the present process comprises pretreatment of the biomass containing matter derived from *Pennisetum* or *Pennisetum hybridum* with hot water, ozone, alkaline solutions, peroxide, an organic solvent and/or an acid.

Acid processes may include concentrated and/or dilute acid steps, such as with sulfuric acid, sulfurous acid, hydrochloric acid, phosphoric acid, organic acids, and/or the like. Dilute-acid pretreatment can be performed either for short retention time at high temperature, e.g. less than 15 minutes, preferably less than 10 minutes, e.g. 5 minutes at more than 150 °C, preferably more than 175°C, eg. at about 180 °C, or for a relatively long retention time at lower temperatures, e.g. for 30-90 minutes at 100-140 °C, e.g. 120 °C.

In organosolv pretreatment, a large number of organic or aqueous organic solvents can be used, typically at temperatures of 150-200 °C Organic solvents such as alcohols, esters, ketones, glycols, organic acids, phenols, and ethers can suitably be employed with or without addition of catalysts such as oxalic, salicylic, and acetylsalicylic acid

Alkaline processes may include caustic materials, such as ammonia, calcium hydroxide, calcium oxide, magnesium hydroxide sodium hydroxide, potassium hydroxide, sodium carbonate, potassium carbonate, sodium bicarbonate, potassium bicarbonate, and/or the like. Alkaline pretreatment can be performed at low temperatures but with a relatively long time and high concentration of the base.

Different types of reactors such as batch, percolation, plug flow, countercurrent, and shrinking-bed reactors, are available for performing these chemical pretreatment steps.

Furthermore, in an embodiment several of the aforementioned pretreatment processes may be combined for a synergistic result.

In one particularly preferred embodiment of the invention the feedstock slurry is subjected to thermal hydrolysis prior to step (iiib) as defined above.

After pretreatment, the feedstock slurry is incubated under conditions suitable for thermophilic or mesophilic fermentation. The term fermentation generally refers to processes in which desired products are formed by the aerobic or anaerobic metabolism of microorganisms. In the method according to the invention the fermentation involves anaerobic digestion of the feedstock slurry using methane producing Euryarchaeota.

Methane-producing Euryarchaeota (also known as methanogens or methanogenic bacteria) constitute a unique group of prokaryotes, which are capable of forming methane from certain classes of organic substrates, methyl substrates (methanol, methylamine, dimethylamine, trimethylamine, methylmercaptan and dimethylsulfide) or acetate (sometimes termed acetoclastic substrate) under anaerobic conditions. Methanogens are found within various genera of bacteria, and methanogenic bacteria of relevance in the context of the present invention include species of *Methanobacterium, Methanobrevibacter, Methanothermus, Methanococcus, Methanomicrobium, Methane genium, Methanospirillum, Methanoplanus, Methanosphaera, Methanosarcina, Methanolobus, Methanoculleus, Methanothrix, Methanosaeta, Methanopyrus* or *Methanocorpusculum;* some of these, notably species of *Methanopyrus,* are highly thermophilic and can grow at temperatures in excess of 100 °C. Only three genera ofmethanogenic bacteria, viz. *Methanosarcina, Methanosaeta* and *Methanothrix,* appear to contain species capable of carrying out the acetoclastic reaction, i.e. conversion of acetate to methane (and carbon dioxide).

It will be appreciated that useful methanogenic bacteria can be selected from a genetically modified bacterium of one of the above useful organism having, relative to the organism from which it is derived, an increased or improved methane producing activity. As used herein the expression "genetically modified bacterium" is used in the conventional meaning of that term i.e. it refers to strains obtained by subjecting a organism to any conventionally used mutagenization treatment including treatment with a chemical mutagen such as ethanemethane sulphonate (EMS) or N-methyl-N'-nitro-N-nitroguanidine (NTG), UV light or to spontaneously occurring mutants, including classical mutagenesis. Furthermore, as it is possible to provide the genetically modified bacterium by random mutagenesis or by selection of spontaneously occurring mutants, i.e. without the use of recombinant DNA-technology, it is envisaged that mutants of the above mentioned organism can be provided by such technology including site-directed mutagenesis and PCR techniques and other in vitro or in vivo modifications of specific DNA sequences once such sequences have been identified and isolated.

In the context of the present invention it will generally be most appropriate to apply, in addition to one or more methanogens, other types of microorganisms which, alone or in combination, are capable of degrading organic substances present in the material to be treated in the anaerobic fermentation step of the process of the invention, but which are not directly suited as substrates for the methanogen(s) employed in the anaerobic fermentation step. Such other types of microorganisms include certain fermentative anaerobic bacteria capable of converting, for example, glucose to products such as acetate, propionate, butyrate, hydrogen and $CO_2$, and so-called acetogenic bacteria, which convert organic substances such as propionate, butyrate and ethanol to acetate, formate, hydrogen and $CO_2$

Fermentation is typically performed by inoculation of the feedstock slurry with the methanogens and subsequently incubating the feedstock slurry at the appropriate temperature.

Inoculation refers to the introduction of one or more microbial isolates in a sufficient concentration to anaerobically digest the feedstock slurry and produce methane gas. A representative microbial inoculum contains both acid forming bacteria and methanogenic bacteria. The microbial isolates in the inoculum may be obtained from a cultured source (e.g., bioreactor) or from a natural source (preexisting soil, decomposing material or semi-liquid manure). The inoculum may also contain genetically engineered microorganisms to perform a specific function, for example, expressing recombinant cellulases to break-down cellulose.

In one embodiment of the invention, undigested feedstock slurry ('raw' slurry) is mixed with slurry that has already been subjected to the fermentation step ('matured' slurry) in order to achieve inoculation with the methanogenic bacteria. An advantage associated with this embodiment is that the matured slurry contains bacteria native to the digesting tank which are already adapted to the conditions within the digesting tank, and should therefore be efficient in digesting the raw slurry. The mixing can be carried out in any suitable way, such as stirring the mixture in a mixing tank or channeling the mixed slurry through a mixer. The mixing can be carried out either within the digesting tank or outside of the digesting tank. To reduce the loss of heat, mixing may be carried out within the digesting tank.

In accordance with the invention, step (iiia) involves thermophilic fermentation, which generally occurs at temperatures up to 70 °C and optimally around 46-60 °C with thermophiles are the primary microorganisms present. In a preferred embodiment step iiia comprises incubation in the presence of a thermophilic methanogen selected from the group consisting of the genus *Methanosarcina,* the genus *Methanosaeta,* Preferably, during step (iiia), the temperature range is controlled to be from 50 °C -57 °C, more preferably from 54 °C - 56 °C, e.g. 55 °C.

In accordance with the invention step (iiib) involves mesophilic fermentation, which generally can occur at ambient temperatures to temperatures up to about 45 °C. Preferably, during step (iiia), the temperature range is controlled to be from 20 °C -40 °C, more preferably from 30 °C - 39 °C, most preferably 35 °C - 39 °C, e.g.37 °C.

The composition in the anaerobic digester is kept at a pH level suitable for the methane production from the waste by the thermophilic or mesophilic methanogenic micoroorganisms. Preferably, the pH of the composition is at least about 7.0 and no more than the maximum pH to produce methane from said composition for the particular constituents in the feedstock slurry. More preferably, the pH is between 7.0 and about 7.5. At least in part, the control of the pH may be done by the control of feed to the digester.

It is typically sufficient to subject the feedstock slurry to the process of step iiia or iiib for a period of time in the range of from10 to 100 days, preferably for a period of time within the range of from 20-60 days. For example, satisfactory methane yield may require incubation for a period of about 50 days. Under appropriate conditions, as will be demonstrated in more detail in the appended examples.

A variety of digesters are suitable for incubating the feedstock slurry during anaerobic production of biogas. Suitable digesters include complete mix digesters, plug-flow digesters, mix and hybrid digesters, fixed film digester, upright cylinder digester, solid state stratified bed (SSB) digester, stirred tank reactor (STR), and batch reactors. A process according to any one of the preceding claims, wherein step (iiia) or (iiib) is performed as a batch or a continuous process in a closed reaction vessel employing mix or plug flow technology. Based on the biogas yield potential of the plant material used, volume or amount of the plant material and the inoculum characteristics, the size of the reactor is calculated. Design characteristics of a biogas reactor based on anaerobic digestion are commercially available and are known to those skilled in the art.

In order for anaerobic digestion to occur, oxygen concentration in the reactor is kept at a minimum preferably at zero. This is done for example by ensuring that the digesting tank is hermetically sealed, and preferably kept under a slight vacuum. This is achieved by extracting gases from the top of the digesting tank (where produced gases accumulate). In addition, it may be possible, in one embodiment, to have a digesting tank in which the reaction chamber is adapted to maintain a slight negative pressure. This may be achieved by hermetically sealing the digesting tank with the aid of a flat or dome-shaped lid having incorporated therein a gas outlet from which gases produced from the digestion may be continuously removed. Alternatively, it is also possible to continuously introduce an inert gas, such as nitrogen, into the digesting tank in order to reduce the amount of oxygen.

Typically, the operation of the digester takes into consideration coordination of the temperature and pH levels of the digesting slurry. The sensing of these parameters may be by direct collection and analysis of samples and/or by the use of strategically positioned sensing probes for collecting data for computerized analysis. A consideration is that as either the pH or the temperature of the slurry increases, the unwanted generation of ammonia may increase. For example, an increase in pH may require the lowering of temperature to maintain the desired methanogen production, or vice versa. A third control parameter can also be the mass feed rate of the slurry, particularly as such affects the concentration of solids in the digester. Make-up water may be required as well and should be considered a component of this parameter. Preferably, the feed rate of waste into the digester, the digester temperature, and the pH of the digester are effectively controlled to produce the desired methanogen production, methane production and ammonia production.

The digester composition is preferably agitated to produce a reaction mixture of gaseous product, e.g. primarily methane, and a pumpable slurry. The pumpable slurry preferably is easily mixed toward a state of homogeneity such that the digester methanogens and the suspended solids interact so that the suspended solids can act as a substrate for the methanogens. Well mixing of the digester liquid phase is also important in accurately sensing the reaction parameters to control and optimize the digesting process.

The digestion of the slurry produces biogas, a large percentage of which comprises methane gas. The methane gas is typically vented from the digesting tank via an outlet arranged at the upper region. In this context, the term biogas refers to the mixture of gases extracted from the outlet of the digesting tank. These gases are derived from a myriad of processes occurring within the reaction chamber, including, respiration, anaerobic fermentation and the production of alcohols and hydrogen by various types of bacteria acting on the slurry. In a preferred embodiment of the invention, a process as defined in any of the foregoing is provided, wherein the methane content in the evolving biogas is at least 30%, preferably at least 40%, more preferably at least 45%, e.g. about 50%, of the total biogas volume. Methane content may easily approach 65% or 70% of the total biogas volume.

In a preferred embodiment of the invention, a process as defined in any of the foregoing is provided, wherein the methane production/yield exceeds 255 $m^3$ per kg of dry biomass material, more preferably wherein the methane production/yield exceeds 275 $m^3$ per kg of dry biomass material. In an embodiment, In an embodiment, methane gas is produced at a yield of about 340-355 liters of methane per kilogram of dry biomass. In another embodiment, methane gas is produced at a concentration about 345 liters to about 350 liters of methane per kilogram of dry biomass. A test known as the Hohenheim Biogas yield Test developed at the University of Hohenheim and as described in the Germany VDI 4630 standards guidance was applied to determine the methane yield potential of several *Pennisetum* plant varieties, especially *Pennisetum hybridum.*

A second aspect of the present invention concerns a process for producing syngas from solid biomass material, the process comprising the steps of: (i) providing biomass; (ii) preparing a gasification feedstock by dehydrating the biomass to a dry matter content of > 85%; and subjecting the biomass to a treatment yielding a particulate material; and (iii) subsequently subjecting said gasification feedstock to a gasification process comprising reacting it with a controlled amount of oxygen and/or steam at a temperature exceeding 700 °C; **characterized** in that the biomass is derived from a plant belonging to the genus *Pennisetum.*

In accordance with this second aspect of the invention the biomass at least comprises matter derived from a plant belonging to the genus *Pennisetum,* in particular from a plant belonging to the species *Pennisetum hybridum.* The biomass ued in the gasification process of the invention may also comprise any of the conventional extremely varied sources, including, for example, wood and other plant material, sawdust, coal, nut shells, sewage sludge, leather waste, tyre and plastic waste, municipal refuse or household residual materials, chicken and cattle litter and manure, slaughterhouse waste, paper waste, food waste, sugar cane bagasse, waste oil, petroleum sludge; coal fines, bone waste, agricultural residues and blend of biomass with fossil waste such as petroleum sludge mixed with bone waste, sewage sludge, sawdust or rape-seed meal. It is preferred that at least 50 wt%, more preferably at least 75 wt%, most preferably at least 90wt% of the total solids weight of the biomass is a plant material derived from *Pennisetum.* It is particularly preferred that at least 50 wt%, , more preferably at least 85 wt%, most preferably at least 90wt% of the total solids weight of the biomass is a plant material derived from *Pennisetum hybridum.* Furthermore, the ratio of *Pennisetum* plant material to non- *Pennisetum* plant material typically exceeds 1:1, preferably 2:1, and most preferably 4:1. In a particularly preferred embodiment of the invention, the ratio of *Pennisetum hybridum* plant material to other plant material typically exceeds 1:1, preferably it exceeds 2:1, and most preferably it exceeds 5:1

In order to process the biomass of the invention into a suitable gasification feedstock, the biomass is preferably dried. It is preferred that the biomass, after drying, contain no more than about 20 wt. %, preferably not more that about 15 wt. %, and more preferably no more than about 10 wt. % water, based on the total weight of the biomass after drying.

Furthermore, the biomass is typically subjected to a size reduction step, prior to, during or after drying, in order to reduce it to a size suitable for gasification. It is preferred that the size reduction step produces a feedstock having an average particle size within the range of 1-100mm, preferably within the range of 5-50 mm, and more preferably within the range of 5-10 mm. Non-limiting examples of mechanical size reduction equipment include rotary breakers, roll crushers, jet mills, cryogenic mills, hammermills, impactors, tumbling mills, roller mills, shear grinders, and knife mills. Hammermills are preferred for the practice of the present invention. Biomass fuel consistency is important in gasification in order to achieve continuous flow through the reactor and to provide reliable product gas composition and calorific value for the downstream energy conversion processes. Furthermore, densification of the biomass fuel reduces the reactor size, while shape and size of the densified fuel reduces fluctuations in gas and fuel flow rates as well as the subsequent product quality. Hence, in a preferred embodiment of the invention the size reduced biomass is subsequently briquetted to densify it and/or to improve the flow rate. Standard processes and equipment are available for this purpose. The Gasification relies on chemical processes at elevated temperatures >700°C and with a limited amount of oxygen or air. The thermal gasification processes are highly endothermic chemical reactions. During the gasification process carbonaceous materials in the feedstock are converted into carbon monoxide and hydrogen by reacting the raw material at high temperatures with a controlled amount of oxygen and/or steam to allow some of the organic material to be "burned" to produce carbon monoxide and energy, which drives a second reaction that converts further organic material to hydrogen and additional carbon dioxide. Further reactions occur when the formed carbon monoxide and residual water from the organic material react to form methane and excess carbon dioxide. The reactive contact with steam and/or oxygen typically results in the use of the entire plant and its every component, i.e., cellulose, hemicellulose, lignin, and other minor components, in the same process, so that the biomass is converted quantitatively. Generally, factors that affect the performance of the thermal gasification reactor system include: stoichiometry of reactants, gasification temperature and pressure, heating rate of feedstock, kind of gasifying agents, residence time, feedstock properties, and catalyst or bed additives.

In a preferred embodiment of the invention, the gasification step (iii) is performed at temperatures higher than 700 °C, preferably more than 800 °C, more preferably more than 900 °C. In one embodiment, the temperatures are maintained below 1400 °C, more preferably below 1200 °C, most preferably below 1000 °C. In another embodiment however, the temperature may be maintained at even higher values, for example at temperatures of about 1500 °C in an oxygen blown gasifier. However, operation at such high temperatures requires a lot of energy and use of expensive refractory materials in the gasifier section, which may not be economically favorable.

In one embodiment, the gasification is performed while the gasifier is under pressure. In one embodiment, the gasifier may be operated at a pressure at a range from 30-85 bar, preferably 40-70 bar. In another embodiment, the gasifier is operated at atmospheric pressure.

Any gasifier can be used in the accordance with the present invention. A gasifier essentially is any reaction vessel in which gasification can be carried out. Gasifier configurations particularly suitable for operation on a commercial scale typically include fixed bed, fluidized bed or entrained flow gasifiers or some variation of these. The invention is however not particularly limited in this respect. Entrained flow gasifiers are preferably employed for large-scale gasification operations. Fluidized bed gasifiers are most useful for fuels that form highly corrosive ash that would damage the walls of slagging gasifiers. Biomass fuels generally contain high levels of corrosive ash.

Suitable methods for supplying heat for the gasification typically include an outside source, e.g. hot char recirculation, and/or sensible heat from a gasification agent; reaction heat from oxidization of a part of feedstock; and/or exothermical reaction heat from a non-carbonaceous material such as calcined lime and $CO_2$

In accordance with the invention, essentially only combustible gases (CO and $H_2$), carbon dioxide, and ash will be the final products. The gas mixture obtained is called synthesis gas or syngas, which may e.g. be burned directly in internal combustion engines, used to produce methanol and hydrogen, or converted via the Fischer-Tropsch process into synthetic fuel.

Yet another aspect of the invention, concerns the use of biomass derived from a plant belonging to the genus *Pennisetum* as a feedstock for biogas and/or methane production. Preferably said use involves the materials and processes as defined in any of the foregoing, as will be readily understood.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The invention will be illustrated in more detail in the following examples, which are not intended to limit the scope here of in any way

**EXAMPLES**

The use of Maralfalfa as feedstock for the production of bioenergy through different technologies has been tested on lab-scale in the following specialized and certified laboratories:

- Combustion
  Instituto de Carboquímica - CSIC. Zaragoza, Spain
- Anaerobic digestion

  - Fachhochschule Giessen Friedberg - University of Applied Sciences. Giessen, Germany
  - Bonalytic GmbH. Troisdorf, Germany

- Gasification
  Ankur Scientific Energy Technologies Pvt. Ltd. Gujarat, India

Combustion

The sample of Maralfalfa is composed of 2 kg of the whole plant, cut when still green into pieces 10-12 cm long. The sample contains a high amount of humidity.

Therefore, before proceeding to the combustion, it is stabilized exposing it to the air, in order to reach a balance with the humidity contained in the laboratory. The plant is then cut into smaller pieces in order to enable the analysis.

Loss for humidity stabilization (%): 85%

| Component | Analytic Regulation | Stabilized | Dry | As received |
|---|---|---|---|---|
| Humidity (% on weight) | | 10,44 | 0,00 | 86,57 |
| Ashes (% on weight) | ISO-1171-1976 | 7,75 | 8,65 | 1,16 |
| Volatiles (% on weight) | ISO-5623-1974 | 0,00 | 0,00 | 0,00 |
| Fixed carbon(%onweight) | | 0,00 | 0,00 | 0,00 |
| Carbon (% on weight) | | 40,57 | 45,30 | 6,09 |
| Hidrogen (% on weight) | | 5,85 | 5,23 | 10,39 |
| Nitrogen (% on weight) | | 1,36 | 1,52 | 0,20 |
| Sulphur (% on weight) | | 0,06 | 0,07 | 0,01 |
| HHV UNE (kcal/kg) | UNE 164001 EX | 3.794 | 4.236 | 569 |
| LHV UNE (kcal/kg) | | 3.431 | 3.964 | -461 |

Anerobic digestion

*Material and methods*

As far as the anaerobic digestion process is concerned, two different kinds of tests can be carried out: batch test and continuous fermentation test. Both tests can be carried out either under mesophilic (35 °C - 39 °C) or thermophilic conditions (54 °C - 56 °C).

Batch test

The batch test is a discontinuous test in which organic substrates or co-substrates are subjected to fermentation under defined anaerobic conditions. The test lasts approximately 30 days, until only a small volume of biogas forms.

The batch test was carried out according to the VDI 4630. The batch test provides information regarding:

- Fundamental evaluation of the possible biogas yield and of the anaerobic biological degradability of a material or mixture of materials
- Qualitative appraisal of the speed of anaerobic degradation of the material under investigation
- Qualitative evaluation of the inhibitory effect of the material under investigation in the range of concentration in the test

Fermentation test apparatus may be as specified in DIN 38414 Part 8 or as specified in DIN EN ISO 11734. All the equipment used was adequately gastight, and more specifically it was made of glass. The reactors had a volume of 11 each and their temperature was maintained constant through water baths.

The substrate was mixed with a seeding sludge, which consisted of an inoculum from an agricultural biogas installation, previously freed from coarse contaminating material. In order to ensure that the biological activity of the seeding sludge being used was in order, a substrate whose biogas potential is known was fermented as well in the batch, as a reference sample.

The gas volume produced was measured according to DIN 38414-8, i.e. through a eudiometer tube. The biogas quantity was read off on a daily basis at the beginning of the measurements. Once the daily gas production fell, the frequency of reading also slowed down to once every three days.

The content of methane and of $CO_2$ was not estimated, but measured. Since it changes over time, it was measured 4 times during the fermentation test.

The batch test was conducted as triple determination. The same applies to the reference and zero samples as well.

Continuous fermentation test

In a continuous test the organic substrates or co-substrates are subjected to fermentation under anaerobic conditions, but the test lasts approximately 3 months and new substrate is daily fed to the reactor.

The test was conducted according to the VDI 4630. For this test, the same type of material is used as for the batch test, but additionally in this case the reactors had an automatic mixing system. The additional information provided by this test, which cannot be obtained with the batch test, regards:

- Process stability in reactors which are continuously fed with the material or mixture of materials under investigation
- Biogas yield under practical conditions due to possible negative or positive synergistic effects
- The mono-fermentability of the substrates under process conditions
- The limits of organic loading rate per unit volume.

On a daily basis both the substrate and the inoculums were added on a daily basis. A the beginning of the test the organic load was maintained below 3,5 kg ODM/m3*d (recommended value), but at the end of test the organic load was increased up to values over 5 kg ODM/m3*d, in order to check the tolerance of the process to this factor.

Once per week the following parameters were measured: pH, acid fats, FOS/TAC, Dry Matter and Organic Dry Matter content, NH4-N and short-chain fatty acids.

The biogas was stored in plastic bags connected through a magnetic valve which registered the amount of biogas produced. The content of methane, $CO_2$, the temperature and the pH were additionally measured by online sensors connected to a PC.

*Results*

1. Batch test under mesophilic conditions conducted by Bonalytic laboratory

| DM [g/kg] | oDM [g/kg] | Biogas [Nl/kg FM] | Biogas [Nl/kg DM] | Biogas [Nl/kg oDM] |
|---|---|---|---|---|
| 268,23 | 251,73 | 93,8 | 349,8 | 372,7 |

2. Batch test under mesophilic conditions conducted by Giessen laboratory

| DM [g/kg] | oDM [g/kg] | Biogas [Nl/kg FM] | Biogas [Nl/kg DM] | Biogas [Nl/kg oDM] |
|---|---|---|---|---|
| 247,8 | 229,7 | 96 | 388 | 418 |

3. Continuous fermentation test under mesophilic and thermophilic conditions conducted by Giessen laboratory

| Parameter | Dimensions | Mesophilic | Thermophilic | % |
|---|---|---|---|---|
| Loading | | | | |
| Reactor volume | 1 | 5,48 | 6,99 | -21,53 |
| Daily input | Kg/d | 0,14 | 0,14 | 0 |
| Retention time | D | 39,2 | 49,9 | -21,5 |
| Organic load | Kg oDM/$m^3$d | 5,98 | 4,62 | 29,28 |
| | | | | |
| Fermenter output | | | | |
| Dry Matter | % | 12,96 | 8,49 | 52,6 |
| Loss on ignition | % DM | 71,42 | 82,21 | -13,1 |
| Organic Dry Matter | % | 9,23 | 6,99 | 32,1 |
| pH | | 7,51 | 7,42 | 1,1 |

(continued)

| Parameter | Dimensions | Mesophilic | Thermophilic | % |
|---|---|---|---|---|
| Organic acids | mg/l | 1.144 | 1.127 | 1,6 |
| Ammonium-N | mg/l | 1.380 | 1.420 | -2,8 |
| | mg/g DM | 10,1 | 16,8 | -40,0 |
| | | | | |
| Biogas | | | | |
| Gas production | $m_n^3/(m^3 d)$ | 2,852 | 1,886 | 51,2 |
| Gas yield | $m_n^3/kg$ oDM | 0,481 | 0,411 | 17,1 |
| Gas yield | $m_n^3/kg$ FM | 0,110 | 0,094 | 17,1 |
| Methane content | Vol % | 53,9 | 53,014 | 1,7 |
| Methane production | $m^3\ CH_4/(m^3 d)$ | 1,439 | 0,966 | 49,0 |
| Methane yield oDM | $m_n^3\ CH_4/kg$ oDM | 0,249 | 0,210 | 18,6 |
| Methane yield | $m_n^3\ CH_4/kg$ FM | 0,057 | 0,048 | 18,0 |
| Hydrogen content | ppm | 0 | 0 | 0 |
| | | | | |
| Decomposition | | | | |
| oDM decomposition | % | 59,94 | 69,666 | -14,0 |
| Gas yield oDM | $m_n^3/kg$ oDMdec | 0,788 | 0,573 | 37,5 |
| Methane yield oDM | $m_n^3\ CH_4/kg$ oDM | 0,424 | 0,305 | 39,0 |

Gasification

*Material and methods*

The gasification test was conducted at the testing facilities of the company Ankur Scientific Energy Technologies, located in India. The process of gasification can be carried out in different types of gasifiers, in which the main difference is the flow direction of the biomass as well as the flow direction of the gas produced.

Sample preparation: The sample used for the gasification test was collected when the plant was already partly dry. In order to conduct the gasification test, further drying was necessary to bring down moisture below 20%. The biomass had very low bulk density even after sizing to 10-28 mm pieces and the sized material did not flow properly. Briquetting is necessary for its possible use in the downdraft gasifier.

The test was conducted in a pilot plant representing the process of a downdraft gasifier. The following equipment was used:

1. Silica crucible with lid
2. Beaker tongs, fisher type
3. Furnace, 1200 ° C
4. Oven (250°C)
5. Analytical balance
6. Beaker
7. Metal cylinder (round)
8. Sieves (0.5,1,2,3,5mm)
9. Desiccators

Determination of moisture content: weighing of the sample in an open crucible and place it in oven at 135°C for 2 hours, this duration can be increased by half an hour each till constancy in weight is achieved. Initial weight of wet sample is referred to as 'Wo'. Final weight of dry sample is referred to as 'W1'. The difference between the initial and final weights gives the moisture content of the sample (moisture content (%) = (W0-W1)/W0 x 100).

Determination of volatile matter: Place the oven dried sample in the crucible obtained from moisture content test in the furnace for 6 min at 600° C. Remove the crucible from the furnace and place it in the desiccators for cooling. Now change the temperature setting to 700 °C & again place the sample at 700°C for another 6 min. remove the crucible from furnace and place it in the desiccators for cooling. Weight of the sample without volatile but containing carbon and ash is referred to as 'W2'. The difference of weight between initial & final weight for volatile matter test give the volatile matter present in the biomass (volatile matter (%) = (W1-W2)/W1 x100).

Determination of ash content: The muffle furnace temp is set at 750°C. when the required temperature is achieved; the sample obtained from volatile matter test is kept in the furnace for 2 hours. See that the sample is fully converted into ash or not. If not, put it for another half an hour or until it is converted into ash. Now remove the crucible and place it in the desiccators for cooling and then weigh it. Weight of the sample containing ash only is referred to as 'W3'. Difference of weight between initial and final weights give the ash content (Ash content (%) = (W3/W1) x 100).

$$\text{Determination of fixed carbon: Fixed Carbon } (\%) = 100-(W2+W3) .$$

Determination of ash fusion: Take about 5 gm biomass sample in an Alumina crucible and put it in a high temperature furnace (furnace should be capable to attain 1250°C or more). Set the furnace temperature at 1200°C and keep the sample for 15 minutes at this temperature. Now, allow the furnace to cool down to 300°C. Remove the sample from furnace and observe if ash was melted or not. The ash melting gives the glassy melted beads.

Determination of flowability test: Take a cylinder of diameter Φ250mm x 460 mm LG, Φ350mm x 450mm LG and then fill with the biomass sample. The cylinder is put in in upward position and shows the sample flow in terms of spreading of sample in all direction. The flowability is assessed as 'poor' (flow is not sustained); 'medium' (flow sustained with the help of mechanical agitation or vibration) or good (Smooth flow).

Determination of bulk density: For bulk density, fill the biomass up to known volume into known volume container (e.g. 5 liters) and weigh the container with sample(kg) so that it gives the sample mass. Bulk density (kg/m$^3$) = mass {(sample + beaker) weight - beaker weight}/ volume (total sample taken in beaker). The bulk density is assessed as low (< 100 kg/m$^3$); medium (100- 200 kg/m$^3$) or high >200kg/m$^3$).

Determination of particle size: Take one kilogram of the sample and first pass through the 0.5mm pore size of the sieve and collect sample into one polythene bag and weigh it, according to 0.5mm porosity sieve size take the other porosity size of the sieves 1,2,3 and 5 mm and collect sample and then weigh it. Collected sample weight is converted into gram percentage of the sample so that it gives the actual concentration of the partial size into the sample (<0.5mm; 0.5-1mm; 1-2 mm; 2-3 mm; 3-5 mm; >5 mm).

*Results*

| Parameter | Dimensions | Value |
|---|---|---|
| Moisture content | % on wet basis | 69,58 |
| Ash | % on dry basis | 6,19 |
| Volatile | % on dry basis | 71,86 |
| Fixed carbon | % on dry basis | 21,95 |
| Ash fusion | °C | Slight deformation at 1200 |
| Bulk density | Kg/m$^3$ | 93 |
| Size | mm | 10 to 28 |
| Ignition test | | Bums easily |
| Flow ability | | Does not flow easily. Briquetting or similar pre-treatment would be required |

**EP 2 503 002 A1**

**Claims**

1.  A process for producing methane gas from solid biomass material, the process comprising the steps of:

    (i) providing biomass;
    (ii) preparing a feedstock slurry containing microbially fermentable components by combining the biomass with an aqueous phase and, optionally, subjecting the biomass to a pre-treatment resulting in reduced resistance to enzymatic degradation; and

    (iiia) incubating the feedstock slurry at a temperature of between 46 and 60 °C and under an oxygen depleted atmosphere, in the presence of methane-producing thermophilic microorganisms and collecting the methane gas released from the feedstock slurry; or
    (iiib) incubating said feedstock slurry at a temperature of between 20 and 40 °C and under an oxygen depleted atmosphere, in the presence of methane-producing mesophilic microorganisms and collecting the methane gas released from the feedstock slurry; **characterized in that** the biomass comprises matter derived from a plant belonging to the genus *Pennisetum.*

2.  A process according to claim 1, wherein the biomass is derived from a plant belonging to the species *Pennisetum hybridum.*

3.  A process according to claim 1 or 2, wherein the biomass is derived from the whole parts of the plant, preferably from stem and leaves.

4.  A process according to any one of the preceding claims, wherein the treatment of step (ii) comprises milling, hydrolysis, steam explosion treatment, irradiation, thermal treatment, organosolv process, ozonlysis, wet oxidation and/or biological degradation.

5.  A process according to any one of the preceding claims, wherein the treatment of step (iiia) or (iiib) is performed in the presence of a microorganism selected from the geneses of *Methanobacterium, Methanobrevibacter, Methanothermus, Methanococcus, Methanomicrobium, Methanogenium, Methanospirilum, Methanoplanus, Methanosphaera, Methanosarcina, Methanolobus, Methanoculleus, Methanothrix, Methanosaeta, Methanopyrus* and *Methanocorpusculum.*

6.  A process according to any one of the preceding claims, wherein step (iiia) or (iiib) is performed for a period of time in the range of 10 to 100 days.

7.  A process according to any one of the preceding claims, wherein the methane production/yield exceeds 275 m$^3$ per kg of dry biomass material.

8.  A process for producing syngas from solid biomass material, the process comprising the steps of:

    (i) providing biomass;
    (ii) preparing a gasification feedstock by dehydrating the biomass to a dry matter content > 85% and subjecting the biomass to a treatment yielding a particulate material; and
    (iii) subsequently subjecting said gasification feedstock to a gasification process comprising reacting it with an amount of oxygen and/or steam at a temperature exceeding 700 °C; **characterized in that** the biomass is derived from a plant belonging to the genus *Pennisetum.*

9.  A process according to claim 8, wherein the biomass is derived from a plant belonging to the species *Pennisetum hybridum.*

10. Use of material derived from a plant belonging to the genus *Pennisetum* as a feedstock for biogas, methane and/or syngas production.

11. Use according to claim 10, wherein the biomass is derived from a plant belonging to the species *Pennisetum hybridum.*

13

**EUROPEAN SEARCH REPORT**

Application Number

EP 11 15 9200

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 200971<br>Thomson Scientific, London, GB;<br>AN 2009-N51926<br>XP000002656428,<br>-& CN 101 519 669 A (UNIV NANJING)<br>2 September 2009 (2009-09-02)<br>* the whole document *<br>----- | 1-7 | INV.<br>C12P5/02<br>C10J3/00 |
| X | NIZAMI ABDUL-SATTAR ET AL: "Review of the Integrated Process for the Production of Grass Biomethane",<br>ENVIRONMENTAL SCIENCE AND TECHNOLOGY, AMERICAN CHEMICAL SOCIETY. EASTON, PA, US,<br>vol. 43, no. 22,<br>15 November 2009 (2009-11-15), pages 8496-8508, XP002632660,<br>ISSN: 0013-936X, DOI: 10.1021/ES901533J<br>[retrieved on 2009-10-12]<br>* the whole document *<br>----- | 1-7 | |
| X | STREZOV V ET AL: "Thermal conversion of elephant grass (Pennisetum Purpureum Schum) to bio-gas, bio-oil and charcoal",<br>BIORESOURCE TECHNOLOGY, ELSEVIER BV, GB,<br>vol. 99, no. 17,<br>1 November 2008 (2008-11-01), pages 8394-8399, XP023182759,<br>ISSN: 0960-8524, DOI:<br>10.1016/J.BIORTECH.2008.02.039<br>[retrieved on 2008-04-11]<br>* the whole document *<br>----- | 8-10 | TECHNICAL FIELDS SEARCHED (IPC)<br><br>C12P<br>C10J |

The present search report has been drawn up for all claims

| Place of search | Date of completion of the search | Examiner |
|---|---|---|
| The Hague | 9 August 2011 | Kools, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 15 9200

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2011

| Patent document cited in search report | Publication date | Patent family member(s) | Publication date |
|---|---|---|---|
| CN 101519669 A | 02-09-2009 | NONE | |